# EUROPÄISCHE PATENTSCHRIFT

(11) **EP 3 880 952 B1**
(45) Veröffentlichungstag und Bekanntmachung des Hinweises auf die Patenterteilung: **16.07.2025**
(21) Anmeldenummer: 19805918.0
(22) Anmeldetag: 13.11.2019
(51) Int. Cl.: F02M 21/02, F02D 19/02, F02D 41/00, F02D 41/06, F17C 13/02, G01N 33/22

(54) **VERFAHREN ZUR ERMITTLUNG EINER KRAFTSTOFFQUALITÄT**
METHOD FOR DETERMINING A FUEL QUALITY
PROCÉDÉ DE DÉTERMINATION D'UNE QUALITÉ DE CARBURANT

(30) Priorität: 16.11.2018 DE 102018128917
(43) Veröffentlichungstag der Anmeldung: 22.09.2021
(73) Patentinhaber: VOLKSWAGEN AKTIENGESELLSCHAFT, 38440 Wolfsburg (DE)
(72) Erfinder: BIPPES, Michael, 38112 Braunschweig (DE)
(74) Vertreter: karo IP
(86) Internationale Anmeldenummer: PCT/EP2019/081140
(87) Internationale Veröffentlichungsnummer: WO 2020/099468

(56) Entgegenhaltungen:
- DE-B3- 102016 206 291
- JP-A- H08 246 903
- KR-A- 20110 062 638
- US-A1- 2013 238 217

## Beschreibung

Die Erfindung betrifft ein Verfahren zur Ermittlung einer Kraftstoffqualität. Der Kraftstoff wird gasförmig einer Verbrennungskraftmaschine zugeführt und in einem Druckbehälter zumindest teilweise verflüssigt bevorratet. Der Kraftstoff ist insbesondere LNG oder ein anderes flüssiges Gas, das bedingt durch seinen Dampfdruck und Siedekurve ein Abblasen eines zur Bevorratung des Kraftstoffs verwendeten Druckbehälters nach einer gewissen Standzeit erforderlich macht.

LNG (*"liquefied natural gas*")*,* also verflüssigtes Methangas oder Erdgas wird heute als Alternative zum Dieselkraftstoff im Bereich von Kraftfahrzeugen (z. B. PKW, LKW, aber auch Schifffahrt, etc.) angesehen.

LNG liegt als tiefkaltes, verflüssigtes Gas in isolierten Tank- und Lagerbehältern (Druckbehältern) vor. Steht ein Kraftfahrzeug still, so erfolgt kein Verbrauch an LNG bzw. verdampften Methan, so dass der Druck im Druckbehälter des Kraftfahrzeugs ansteigt. Kurz vor Erreichen des zulässigen Maximaldrucks wird der Druckbehälter über zumindest ein Sicherheitsventil gegen die Atmosphäre entspannt. Dadurch verliert der Druckbehälter an Inhalt und das Kraftfahrzeug an verbleibender Reichweite.

Gerade LNG liegt nicht immer in der gleichen Zusammensetzung vor. Die Gehalte an CH4, CO2, N2 und H2 sowie höheren Kohlenwasserstoffen wie C2H6, C2H4, C3H8 und höhere Kohlenwasserstoffe bis Hexan als unterschiedliche Bestandteile des Kraftstoffes können variieren. Dabei sind Verbrennungskraftmaschinen, die LNG verbrennen, auf die hohe Klopffestigkeit (Eigenschaft eines Kraftstoffes bzw. eines Bestandteils eines Kraftstoffes, sich nicht vorzeitig selbsttätig zu entzünden, z. B. in einem Ottomotor nur aufgrund einer Verdichtung) von CH4 angewiesen. Für den Betrieb einer Verbrennungskraftmaschine ist es insbesondere bedeutsam, die Zusammensetzung eines Kraftstoffes zu kennen.

Aus der DE 10 2005 009 823 A1 ist ein System zur Überwachung eines in einem Druckbehälter bevorrateten komprimierten Gases bekannt.

Aus der DE 10 2007 022 610 A1 ist ein Druckgas-Speichersystem mit integrierter Druckentlastungsvorrichtung bekannt.

Die DE 10 2012 024 717 A1 ist auf ein Fahrzeug mit einem Flüssiggastank und einer Überdruckablassleitung gerichtet.

In der DE 601 31 484 T2 wird ein Kraftstoffsystem beschrieben, bei dem über eine Boarddiagnose eine Unversehrtheit einer Kraftstoffdampfrückgewinnung überwacht wird.

Aus der DE 602 08 563 T2 ist ein Überwachungssystem für einen Druckbehälter bekannt.

Aus der KR 2011 0062638 A ist eine Kraftstofftankanordnung bekannt, bei der der Anteil an abgeführtem Kraftstoff aus einem Druckbehälter über eine Sicherheitseinrichtung gemessen wird und damit die Qualität des Restkraftstoffes im Druckbehälter bestimmt wird.

Aufgabe der vorliegenden Erfindung ist es, die mit Bezug auf den Stand der Technik angeführten Probleme zumindest teilweise zu lösen. Insbesondere soll ein Verfahren zur Ermittlung einer Kraftstoffqualität vorgeschlagen werden, so dass ein sicherer Betrieb der Verbrennungskraftmaschine gewährleistet werden kann.

Zur Lösung dieser Aufgaben trägt ein Verfahren mit den Merkmalen gemäß Patentanspruch 1 bei. Vorteilhafte Weiterbildungen sind Gegenstand der abhängigen Patentansprüche.

Es wird ein Verfahren zur Ermittlung einer Kraftstoffqualität eines Kraftstoffes vorgeschlagen. Der Kraftstoff wird gasförmig einer Verbrennungskraftmaschine zugeführt, wobei der Kraftstoff ein in einem Druckbehälter bevorrateter, zumindest teilweise verflüssigter, Kraftstoff ist. Der Druckbehälter wird bei Erreichen eines Grenzdruckes (regelmäßig) über eine Sicherheitseinrichtung (z. B. ein Sicherheitsventil oder ähnliches) an eine Umgebung entspannt, indem ein gasförmiger Bestandteil des Kraftstoffes aus dem Druckbehälter entfernt wird. Das Verfahren umfasst zumindest den folgenden Schritt:
a) Ermitteln der Kraftstoffqualität des in dem Druckbehälter bevorrateten Kraftstoffes unter Berücksichtigung zumindest einer noch erfolgenden Abführung eines gasförmigen Bestandteils des Kraftstoffes über die Sicherheitseinrichtung, wobei eine Prognose hinsichtlich einer zu einem zukünftigen Zeitpunkt über die Sicherheitseinrichtung abzuführenden Menge an dem gasförmigen Bestandteil des Kraftstoffes berücksichtigt wird.

Der Kraftstoff ist ein LNG- oder anderer flüssiger Gas-Kraftstoff. Der Kraftstoff wird in einem Druckbehälter bevorratet und zur Verbrennung einer Verbrennungskraftmaschine zugeführt. Die Verbrennungskraftmaschine kann z. B. in einem Kraftfahrzeug zum Antrieb des Kraftfahrzeuges angeordnet sein.

Das Verfahren kann insbesondere in einem Kraftfahrzeug oder auch in einer Anlage zur Lagerung von verflüssigten Gasen eingesetzt werden. Bei der Bevorratung eines Kraftstoffes, z. B. von LNG in Kraftfahrzeugen, können insbesondere sich leichter verflüchtigende Bestandteile des Kraftstoffes zu höheren Anteilen über die Sicherheitseinrichtung (Überdruckventil, Sicherheitsventil) abgeführt werden, so dass sich die Zusammensetzung des im Druckbehälter verbleibenden Kraftstoffes verändern kann. Bedingt durch seine Stoffeigenschaften kann insbesondere CH4 aus dem Gemisch verschiedener Bestandteile des Kraftstoffes als erstes bzw. zu höheren Anteilen verdampfen und damit zum Druckaufbau in einer Gasphase in einem Druckbehälter beitragen.

Beim zyklisch erforderlichen Entspannen des Druckbehälters bzw. des Kraftstoffes wird damit ggf. überproportional dieser Bestandteil, also z. B. CH4, an die Umgebung abgeführt. Damit wird die Konzentration bzw. der Anteil der anderen Bestandteile und insbesondere der anderen Kohlenwasserstoffe im verbleibenden flüssigen Kraftstoff (bzw. Kraftstoffgemisch) ansteigen. Insbesondere können so die Stoffeigenschaften des Kraftstoffes, insbesondere im Hinblick auf eine Klopffestigkeit, verändert werden. Ein Absinken der Klopffestigkeit kann jedoch zu den allgemein bekannten Problemen bei der Verbrennung des Kraftstoffes führen und erfordert insbesondere entsprechende Gegenmaßnahmen, wie z. B. eine Anpassung der Einspritzmenge.

Mithilfe des vorgeschlagenen Verfahrens ist es nun insbesondere möglich, die veränderte Zusammensetzung des für die Verbrennung in der Verbrennungskraftmaschine vorgesehenen Kraftstoffes zu ermitteln und die die Verbrennung beeinflussenden Parameter darauf einzustellen. Insbesondere wird die Kraftstoffqualität vor einer Inbetriebnahme einer Verbrennungskraftmaschine und vor der Durchführung eines Verbrennungsvorgangs des Kraftstoffes ermittelt.

Insbesondere wird so eine vorrausschauende Ermittlung der Kraftstoffqualität durchgeführt. Damit kann ein Nutzer des Kraftfahrzeuges z. B. direkt am Kraftfahrzeug oder telemetrisch darüber informiert werden, dass die Kraftstoffqualität nicht mehr zum regelgerechten Betrieb der Verbrennungskraftmaschine ausreicht oder insbesondere die Verbrennungskraftmaschine nicht mehr in Betrieb genommen werden sollte. Damit kann insbesondere eine Beschädigung des Kraftfahrzeugs durch ungenügende Kraftstoffqualität, z. B. nach längeren Standzeiten des Kraftfahrzeuges, verhindert werden.

Telemetrisch (bzw. telemetrisch übermittelt) heißt insbesondere, dass Informationen (Kraftstoffqualität, etc. oder auch Datensätze) zu einer räumlich getrennten Empfangsstelle (z. B. über Funk) übermittelt werden (z. B. zum Kraftfahrzeug oder auf ein Mobiltelefon, einen Rechner, etc.).

Datensätze können z. B. in einer Steuereinheit am Druckbehälter oder im Kraftfahrzeug ermittelt und an der Empfangsstelle zumindest angezeigt, gesammelt und aufgezeichnet oder auch ausgewertet werden.

Bevorzugt wird die Kraftstoffqualität bei dem Betrieb einer Verbrennungskraftmaschine berücksichtigt. Insbesondere werden also die die Verbrennung beeinflussenden Parameter unter Berücksichtigung der Kraftstoffqualität verändert bzw. angepasst (z. B. Einspritzmenge, Einspritzzeitpunkt, Zündzeitpunkt, Gemischbildung, Verdichtung usw.).

Bevorzugt wird für die Kraftstoffqualität zumindest eine Klopffestigkeit des Kraftstoffes ermittelt.

Insbesondere wird unter Berücksichtigung der Kraftstoffqualität eine Inbetriebnahme der Verbrennungskraftmaschine verhindert.

Insbesondere wird für Schritt a) eine anhand eines Verbrennungsvorgangs in der Verbrennungskraftmaschine bestimmte Kraftstoffqualität berücksichtigt.

Insbesondere wird z. B. zumindest einer der folgenden Parameter (des Kraftstoffes oder der Verbrennungskraftmaschine, z. B. eine durch den Kraftstoff bedingte Eigenschaft der Verbrennungskraftmaschine) anhand eines Verbrennungsvorgangs (oder mehrerer Verbrennungsvorgänge) bestimmt: Energiegehalt des Kraftstoffes; Methanzahl des Kraftstoffes; Leistung der Verbrennungskraftmaschine; Verhältnis Wasserstoff zu Kohlenstoff, (z. B. ermittelt durch Luftmassenmessung und Lambda-Sonde), Zusammensetzung Abgas (Anteile CO2 und/oder O2). Diese Daten können insbesondere zur Verifizierung bzw. Kalibrierung der gemäß Schritt a) ermittelten Kraftstoffqualität verwendet werden.

Weiter kann für Schritt a) eine Angabe hinsichtlich des in den Druckbehälter eingeführten Kraftstoffes berücksichtigt werden. Diese Angabe kann z. B. manuell eingegeben oder telemetrisch (z. B. an ein Steuergerät) übermittelt werden. Die Angabe kann zumindest einen der folgenden Parameter des Kraftstoffes umfassen: Methanzahl, Energiegehalt, Methangehalt, etc. Insbesondere kann die Angabe bei einem Betankungsvorgang des Druckbehälters übermittelt werden.

Insbesondere kann die tatsächliche bzw. vermutete, ermittelte oder verifizierte Zusammensetzung des Kraftstoffes bei dem Verfahren berücksichtigt werden. Diese Kenntnis der Zusammensetzung ermöglicht eine genauere Prognose des Verdampfungsverhaltens sowie eines resultierenden Druckes der Gasphase und der über die Sicherheitseinrichtung abgegebenen Menge an Kraftstoff (bzw. Methan also CH4).

Die tatsächliche Zusammensetzung kann durch das Kraftfahrzeug selber z. B. mittels einer Vorrichtung zur Bestimmung der aktuellen Zusammensetzung des Kraftstoffs im Druckbehälter selbst oder in kraftstoffführenden Bauteilen erfolgen. Weiter kann die Bestimmung durch Auswertung des Verbrennungsverhaltens des Kraftstoffes in der Verbrennungskraftmaschine oder durch eine Übertragung der Kraftstoffqualität von einer Betankungsanlage mit den bekannten Kraftstoffqualitäten erfolgen. Auch eine manuelle Eingabe der Kraftstoffqualität kann erfolgen. Ggf. ist eine Kombination der genannten Möglichkeiten, insbesondere zur Plausibilisierung, vorteilhaft.

Insbesondere wird ein Kraftstoff bzw. dessen Zusammensetzung bestimmt. Insbesondere kann ausgehend von der bestimmten Zusammensetzung ein Modell berücksichtigt werden, das die Verdampfung bzw. die Zusammensetzung des Dampfes beschreibt. Insbesondere kann der Kraftstoff bzw. dessen Zusammensetzung mit Hilfe von Sensorik, wie z. B. Massendurchflussmesser bzw. -messung und/oder druck- und temperaturkompensierte Durchflussmessung, bestimmt werden.

Insbesondere wird für Schritt a) zumindest eine der folgenden Umgebungsbedingungen berücksichtigt:
- eine klimatische Umgebungsbedingung (Luftfeuchtigkeit, Lufttemperatur, Luftdruck, prognostizierte Änderung der Umgebungsbedingung, etc.) des Druckbehälters,
- eine geographische Umgebungsbedingung (Örtlichkeit, also z. B. Fähre, Parkhaus, Tunnel, Wohngebiet, etc.; Höhe in Relation zu Normal Null, Sonneneinstrahlung, etc.; Land bzw. Staat; prognostizierte Änderung der Umgebungsbedingung z. B. infolge einer Fortbewegung des Kraftfahrzeuges, ggf. unter Berücksichtigung eines Navigationssystems, etc.) des Druckbehälters,

- eine am Ort des Druckbehälters vorliegende gesetzliche Vorschrift,
- eine durch eine Sensorik des Druckbehälters bzw. des Kraftfahrzeugs (Kamera, Radar, Ultraschall) ermittelte Umgebungsbedingung des Druckbehälters (Berücksichtigung von Lebewesen, Bauten, geschlossenen Räumen, etc. in der Umgebung des Kraftfahrzeuges).

Außerdem wird erfindungsgemäß für Schritt a) eine Prognose hinsichtlich einer zu einem zukünftigen Zeitpunkt über die Sicherheitseinrichtung abzuführenden Menge an gasförmigen Bestandteilen des Kraftstoffes berücksichtigt.

Bei der Prognose hinsichtlich einer zu einem zukünftigen Zeitpunkt über die Sicherheitseinrichtung abzuführenden Menge an Kraftstoff kann insbesondere berücksichtigt werden, dass z. B. der Druckbehälter zu einem bestimmten zukünftigen Zeitpunkt entspannt werden muss. Die Prognose wird eingesetzt, um die (im Druckbehälter verbleibende) Kraftstoffqualität zu einem in der Zukunft liegenden Zeitpunkt vorherzubestimmen (zu prognostizieren) oder die Entwicklung der Kraftstoffqualität des im Druckbehälter verbleibenden Kraftstoffes über der Zeit zu bestimmen.

Insbesondere kann durch Berücksichtigung einer oder mehrerer Umgebungsbedingungen sichergestellt werden, dass die Kraftstoffqualität mit höherer Genauigkeit bestimmt oder vorherbestimmt werden kann. Hinsichtlich der Prognose wird auf die nachveröffentlichte DE 10 2018 209 423.3 verwiesen. Die dort erläuterten Verfahren zur Bestimmung eines Zeitpunkts, an dem ein Druck im Druckbehälter einen Druckgrenzwert (erster Grenzdruck) erreicht, können vorliegend angewendet werden.

Insbesondere kann für Schritt a) zumindest einer der folgenden Parameter berücksichtigt werden: Temperatur im Druckbehälter, Druck im Druckbehälter, Betankungsmenge bzw. Füllstand des Druckbehälters.

Es wird weiter ein Kraftfahrzeug vorgeschlagen, zumindest aufweisend eine mit einem gasförmigen Kraftstoff betreibbare Verbrennungskraftmaschine und einen Druckbehälter zur Bevorratung des zumindest teilweise verflüssigten Kraftstoffes (LNG oder anderer flüssiger Gas-Kraftstoff) sowie eine Steuereinheit, die zur Durchführung des beschriebenen Verfahrens eingerichtet ist. Der Druckbehälter weist eine Sicherheitseinrichtung auf zum Entspannen des Druckbehälters an eine Umgebung. Das Steuergerät ist zur Durchführung des Verfahrens geeignet ausgeführt bzw. führt das Verfahren aus. Die Verbrennungskraftmaschine kann durch die Steuereinheit in Betrieb genommen werden und deren Betrieb ist durch die Steuereinheit regelbar. Weiter kann das Verfahren auch von einem Computer bzw. mit einem Prozessor einer Steuereinheit ausgeführt werden.

Es wird demnach auch ein System zur Datenverarbeitung (insbesondere ein Steuergerät bzw. Teil davon) vorgeschlagen, das einen Prozessor umfasst, der so angepasst/konfiguriert ist, dass er das Verfahren bzw. einen Teil der Schritte des vorgeschlagenen Verfahrens ausführt.

Es kann ein computerlesbares Speichermedium vorgesehen sein, das Befehle umfasst, die bei der Ausführung durch einen Computer/Prozessor diesen veranlassen, das Verfahren bzw. mindestens einen Teil der Schritte des vorgeschlagenen Verfahrens auszuführen.

Die Ausführungen zu dem Verfahren sind insbesondere auf das Kraftfahrzeug, das System, das Speichermedium oder das computerimplementierte Verfahren übertragbar und umgekehrt.

Das Verfahren ermöglicht insbesondere auch, dass ein Nutzer des Kraftfahrzeuges über eine Kraftstoffqualität, eine Veränderung der Kraftstoffqualität, eine (ggf. zu erwartende nur) eingeschränkte Nutzung des Kraftfahrzeuges bzw. eine Warnung hinsichtlich der Inbetriebnahme der Verbrennungskraftmaschine informiert werden kann.

Das Verfahren kann insbesondere auch außerhalb von Kraftfahrzeugen z. B. zur Überwachung von Druckbehältern bzw. Anlagen zur Lagerung von flüssigen Gasen eingesetzt werden.

Vorsorglich sei angemerkt, dass die hier verwendeten Zahlwörter ("erste", "zweite", ...) vorrangig (nur) zur Unterscheidung von mehreren gleichartigen Gegenständen, Größen oder Prozessen dienen, also insbesondere keine Abhängigkeit und/oder Reihenfolge dieser Gegenstände, Größen oder Prozesse zueinander zwingend vorgeben. Sollte eine Abhängigkeit und/oder Reihenfolge erforderlich sein, ist dies hier explizit angegeben oder es ergibt sich offensichtlich für den Fachmann beim Studium der konkret beschriebenen Ausgestaltung.

Die Erfindung sowie das technische Umfeld werden nachfolgend anhand der beiliegenden Figuren näher erläutert. Es ist darauf hinzuweisen, dass die Erfindung durch die angeführten Ausführungsbeispiele nicht beschränkt werden soll. Insbesondere ist darauf hinzuweisen, dass die Figuren und insbesondere die dargestellten Größenverhältnisse nur schematisch sind. Es zeigen:
- Fig. 1:: ein Kraftfahrzeug;
- Fig. 2:: ein erstes Diagramm; und
- Fig. 3:: ein zweites Diagramm.

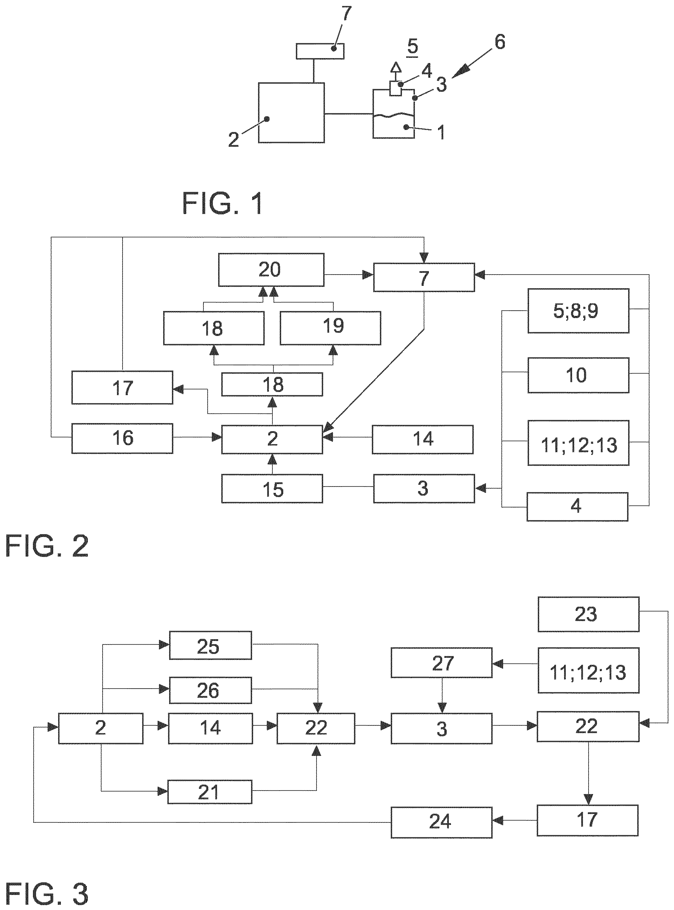

Fig. 1 zeigt ein Kraftfahrzeug 6 mit einer mit einem gasförmigen Kraftstoff 1 betreibbaren Verbrennungskraftmaschine 2 und einem Druckbehälter 3 zur Bevorratung des zumindest teilweise verflüssigten Kraftstoffes 1 sowie einer Steuereinheit 7, die zur Durchführung des beschriebenen Verfahrens eingerichtet ist. Der Druckbehälter 3 weist eine Sicherheitseinrichtung 4 zum Entspannen des Druckbehälters 3 an eine Umgebung 5 auf.

Fig. 2 zeigt ein erstes Diagramm. Darin ist ein erster Ablauf des Verfahrens dargestellt. In dem Druckbehälter 3 kann eine Kraftstoffqualität des darin bevorrateten Kraftstoffes 1 anhand mehrerer Paramater ermittelt werden. Diese Parameter umfassen z. B. eine erste Temperatur 8 und einen ersten Druck 9 einer Umgebung 5, die Erfassung einer Betankungsmenge 10, die Erfassung eines Füllstands 11, eines zweiten Drucks 12 und einer zweiten Temperatur 13 innerhalb des Druckbehälters 3 sowie die Erfassung einer über die Sicherheitseinrichtung 4 abgegebenen Menge und ggf. Kraftstoffqualität des gasförmigen Kraftstoffes 1.

Die Verbrennungskraftmaschine 2 kann hinsichtlich der Parameter Leistung 14, Verbrauch 15 an Kraftstoff 1, Zuluftmenge 16 überwacht werden. Weiter kann die Klopffestigkeit 17 der Verbrennungsvorgänge überprüft werden.

Weiter können die Parameter des Abgases 18 überprüft werden, z. B. über die Erfassung der CO2-Konzentration 18, der O2-Konzentration 19 und (ggf. daraus hergeleitet) über die Ermittlung des Wasserstoff/Kohlenstoff-Verhältnisses 20.

Die beschriebenen Parameter können über die Steuereinheit 7 ausgewertet werden.

Fig. 3 zeigt ein zweites Diagramm. Darin ist ein zweiter Ablauf des Verfahrens dargestellt.

In dem Druckbehälter 3 wird eine über die Sicherheitseinrichtung 4 abgegebene Menge und ggf. Kraftstoffqualität des gasförmigen Kraftstoffes 1 (Erfassung Boil-Off 27) bestimmt, unter Berücksichtigung von zweitem Druck 12 und zweiter Temperatur 13 innerhalb des Druckbehälters 3 sowie des Füllstands 11. Weiter wird eine Angabe 23 hinsichtlich des in den Druckbehälter 3 eingeführten Kraftstoffes 1 berücksichtigt, z. B. von einer Betankungsanlage. Davon ausgehend werden neue Eigenschaften 22 bzw. die neue Zusammensetzung des Kraftstoffes 1 bestimmt.

Anhand dieser neuen Eigenschaften 22 wird eine Klopffestigkeit 17 bestimmt. Diese Klopffestigkeit 17 wird für den Betrieb bzw. die Inbetriebnahme der Verbrennungskraftmaschine 2 berücksichtigt. Anhand der Klopffestigkeit 17 wird also eine Entscheidung 24 zum Betrieb der Verbrennungskraftmaschine 2 getroffen.

Anhand der in Betrieb genommenen Verbrennungskraftmaschine 2 können die Parameter Energiegehalt 25, Methanzahl 26, Leistung 14 und Wasserstoff/Kohlenstoff-Verhältnis 21 bestimmt werden, die zur weiteren Bestimmung bzw. Verifizierung/Plausibilisierung/Kalibrierung der Kraftstoffqualität bzw. der Eigenschaften 22 des Kraftstoffes 1 verwendet werden können.

Die so gewonnenen Erkenntnisse können dann im Zusammenhang mit den im Druckbehälter 3 erfassten Parameter weiterverwendet werden.

### Bezugszeichenliste

- 1: Kraftstoff
- 2: Verbrennungskraftmaschine
- 3: Druckbehälter
- 4: Sicherheitseinrichtung
- 5: Umgebung
- 6: Kraftfahrzeug
- 7: Steuereinheit
- 8: erste Temperatur
- 9: erster Druck
- 10: Betankungsmenge
- 11: Füllstand
- 12: zweiter Druck
- 13: zweite Temperatur
- 14: Leistung
- 15: Verbrauch
- 16: Zuluftmenge
- 17: Klopffestigkeit
- 18: Abgas
- 19: CO2-Konzentration
- 20: O2-Konzentration
- 21: Wasserstoff/Kohlenstoff-Verhältnis
- 22: neue Eigenschaft
- 23: Angabe
- 24: Entscheidung
- 25: Energiegehalt
- 26: Methanzahl
- 27: Boil-Off

## Patentansprüche

1. Verfahren um eine Kraftstoffqualität eines Kraftstoffes (1), der gasförmig einer Verbrennungskraftmaschine (2) zugeführt wird, zu einem in der Zukunft liegenden Zeitpunkt vorherzubestimmen oder die Entwicklung der Kraftstoffqualität des in einem Druckbehälter (3) verbleibenden Kraftstoffes (1) über der Zeit zu bestimmen, wobei der Kraftstoff (1) ein in dem Druckbehälter (3) bevorrateter, zumindest teilweise verflüssigter, Kraftstoff (1) ist, wobei der Druckbehälter (3) bei Erreichen eines Grenzdruckes über eine Sicherheitseinrichtung (4) an eine Umgebung (5) entspannt wird, indem ein gasförmiger Bestandteil des Kraftstoffes (1) aus dem Druckbehälter (3) entfernt wird; wobei das Verfahren zumindest den folgenden Schritt umfasst:
a) Ermitteln der Kraftstoffqualität des in dem Druckbehälter (3) bevorrateten Kraftstoffes (1) unter Berücksichtigung zumindest einer noch erfolgenden Abführung eines gasförmigen Bestandteils des Kraftstoffes (1) über die Sicherheitseinrichtung (4), wobei eine Prognose hinsichtlich einer zu einem zukünftigen Zeitpunkt über die Sicherheitseinrichtung (4) abzuführenden Menge des gasförmigen Bestandteils des Kraftstoffes (1) berücksichtigt wird.

2. Verfahren nach Patentanspruch 1, wobei die Kraftstoffqualität vor einer Inbetriebnahme einer Verbrennungskraftmaschine (2) und vor der Durchführung eines Verbrennungsvorgangs des Kraftstoffes (1) ermittelt wird.

3. Verfahren nach einem der vorhergehenden Patentansprüche, wobei die Kraftstoffqualität bei dem Betrieb einer Verbrennungskraftmaschine (2) berücksichtigt wird.

4. Verfahren nach einem der vorhergehenden Patentansprüche, wobei für die Kraftstoffqualität eine Klopffestigkeit des Kraftstoffes (1) ermittelt wird.

5. Verfahren nach einem der vorhergehenden Patentansprüche, wobei unter Berücksichtigung der Kraftstoffqualität eine Inbetriebnahme der Verbrennungskraftmaschine (2) verhindert wird.

6. Verfahren nach einem der vorhergehenden Patentansprüche, wobei für Schritt a) eine anhand eines Verbrennungsvorgangs in der Verbrennungskraftmaschine (2) bestimmte Kraftstoffqualität berücksichtigt wird.

7. Verfahren nach einem der vorhergehenden Patentansprüche, wobei für Schritt a) eine Angabe hinsichtlich des in den Druckbehälter (3) eingeführten Kraftstoffes (1) berücksichtigt wird.

8. Verfahren nach einem der vorhergehenden Patentansprüche, wobei für Schritt a) zumindest eine der folgenden Umgebungsbedingungen berücksichtigt wird:
• eine klimatische Umgebungsbedingung des Druckbehälters (3),
• eine geographische Umgebungsbedingung des Druckbehälters (3),
• eine am Ort des Druckbehälters (3) vorliegende gesetzliche Vorschrift,
• eine durch eine Sensorik des Druckbehälters (3) ermittelte Umgebungsbedingung des Druckbehälters (3).

9. Kraftfahrzeug (6), zumindest aufweisend eine mit einem gasförmigen Kraftstoff (1) betreibbare Verbrennungskraftmaschine (2) und einen Druckbehälter (3) zur Bevorratung des zumindest teilweise verflüssigten Kraftstoffes (1), wobei der Druckbehälter (3) eine Sicherheitseinrichtung (4) zum Entspannen des Druckbehälters (3) an eine Umgebung (5) aufweist, sowie eine Steuereinheit (7), die zur Durchführung eines Verfahrens nach einem der vorhergehenden Patentansprüche eingerichtet ist; wobei die Verbrennungskraftmaschine (2) durch die Steuereinheit (7) inbetriebnehmbar und deren Betrieb durch die Steuereinheit (7) regelbar ist.

## Claims

1. Method for predicting a fuel quality of a fuel (1), which is supplied in gaseous form to an internal combustion engine (2), at a future time or determining the development of the fuel quality of the fuel (1) remaining in a pressure vessel (3) over time, wherein the fuel (1) is an at least partially liquefied fuel (1) stored in the pressure vessel (3), wherein, when a limit pressure is reached, the pressure vessel (3) is depressurized to an environment (5) via a safety device (4) by way of a gaseous component of the fuel (1) being removed from the pressure vessel (3); wherein the method comprises at least the following step:
(a) determining the fuel quality of the fuel (1) stored in the pressure vessel (3), taking into account at least one ongoing discharge of a gaseous component of the fuel (1) via the safety device (4), wherein a forecast relating to a quantity of the gaseous component of the fuel (1) to be discharged via the safety device (4) at a future time is taken into account.

2. Method according to Claim 1, wherein the fuel quality is determined before commissioning an internal combustion engine (2) and before carrying out a combustion process on the fuel (1).

3. Method according to either of the preceding claims, wherein the fuel quality is taken into account during operation of an internal combustion engine (2).

4. Method according to any of the preceding claims, wherein a knock resistance of the fuel (1) is determined for the fuel quality.

5. Method according to any of the preceding claims, wherein commissioning of the internal combustion engine (2) is prevented taking into account the fuel quality.

6. Method according to any of the preceding claims, wherein a fuel quality determined on the basis of a combustion process in the internal combustion engine (2) is taken into account for step a).

7. Method according to any of the preceding claims, wherein an indication relating to the fuel (1) introduced into the pressure vessel (3) is taken into account for step a).

8. Method according to any of the preceding claims, wherein at least one of the following environmental conditions is taken into account for step a):
• a climatic environmental condition of the pressure vessel (3),
• a geographical environmental condition of the pressure vessel (3),
• a legal requirement applicable at the site of the pressure vessel (3),
• an environmental condition of the pressure vessel (3) determined by a sensor system of the pressure vessel (3).

9. Motor vehicle (6), at least comprising an internal combustion engine (2) which can be operated with a gaseous fuel (1) and a pressure vessel (3) for storing the at least partially liquefied fuel (1), wherein the pressure vessel (3) has a safety device (4) for depressurizing the pressure vessel (3) to an environment (5), and a control unit (7), which is designed for carrying out a method according to any of the preceding claims; wherein the internal combustion engine (2) can be commissioned by the control unit (7) and operation of the internal combustion engine can be controlled by the control unit (7).

## Revendications

1. Procédé pour définir au préalable une qualité de carburant d'un carburant (1), qui est amené sous forme gazeuse à un moteur à combustion interne (2), à un moment futur ou pour définir l'évolution de la qualité de carburant du carburant (1) restant dans un contenant sous pression (3) dans le temps, le carburant (1) étant un carburant (1) au moins en partie liquéfié, stocké dans le contenant sous pression (3), le contenant sous pression (3) étant détendu dans un environnement (5) par un dispositif de sécurité (4) lorsqu'une pression limite est atteinte, en ce qu'un composant gazeux du carburant (1) est retiré du contenant sous pression (3) ; le procédé comprenant au moins l'étape suivante :
a) la détermination de la qualité de carburant du carburant (1) stocké dans le contenant sous pression (3) en tenant compte d'au moins une évacuation encore en cours d'un composant gazeux du carburant (1) par le dispositif de sécurité (4), une prévision quant à une quantité du composant gazeux du carburant (1) à évacuer par le dispositif de sécurité (4) à un moment futur étant prise en compte.

2. Procédé selon la revendication 1, la qualité de carburant étant déterminée avant une mise en service d'un moteur à combustion interne (2) et avant la mise en œuvre d'une opération de combustion du carburant (1).

3. Procédé selon l'une des revendications précédentes, la qualité de carburant étant prise en compte lors du fonctionnement d'un moteur à combustion interne (2).

4. Procédé selon l'une des revendications précédentes, un pouvoir antidétonant du carburant (1) étant déterminé pour la qualité de carburant.

5. Procédé selon l'une des revendications précédentes, une mise en service du moteur à combustion interne (2) étant empêchée en tenant compte de la qualité de carburant.

6. Procédé selon l'une des revendications précédentes, une qualité de carburant définie à l'aide d'une opération de combustion dans le moteur à combustion interne (2) étant prise en compte pour l'étape a).

7. Procédé selon l'une des revendications précédentes, une indication concernant le carburant (1) introduit dans le contenant sous pression (3) étant prise en compte pour l'étape a).

8. Procédé selon l'une des revendications précédentes, au moins une des conditions d'environnement suivantes étant prise en compte pour l'étape a) :
• une condition d'environnement climatique du contenant sous pression (3),
• une condition d'environnement géographique du contenant sous pression (3),
• une disposition légale en vigueur sur le lieu du contenant sous pression (3),
• une condition d'environnement du contenant sous pression (3) déterminée par un équipement de détection du contenant sous pression (3).

9. Véhicule à moteur (6), comportant au moins un moteur à combustion interne (2) pouvant fonctionner avec un carburant (1) gazeux et un contenant sous pression (3) pour stocker le carburant (1) au moins en partie liquéfié, le contenant sous pression (3) comportant un dispositif de sécurité (4) pour détendre le contenant sous pression (3) dans un environnement (5), ainsi qu'une unité de commande (7), qui est mise au point pour mettre en œuvre un procédé selon l'une des revendications précédentes ; le moteur à combustion interne (2) pouvant être mis en service par l'unité de commande (7) et son fonctionnement pouvant être régulé par l'unité de commande (7).
